# EUROPÄISCHE PATENTSCHRIFT

(11) **EP 0 812 153 B1**
(45) Veröffentlichungstag und Bekanntmachung des Hinweises auf die Patenterteilung: **19.05.1999**
(21) Anmeldenummer: 96905781.9
(22) Anmeldetag: 23.02.1996
(51) Int. Cl.: A61B 10/00

(54) **MESSSYSTEM ZUR BESTIMMUNG DER FETTAUSSCHEIDUNG DER HAUT**
MEASURING SYSTEM FOR DETERMINING THE SECRETION OF SEBUM FROM THE SKIN
SYSTEME DE MESURE PERMETTANT DE DETERMINER LA SECRETION DE SEBUM DE LA PEAU

(30) Priorität: 24.02.1995 DE 29503080 U
(43) Veröffentlichungstag der Anmeldung: 17.12.1997
(73) Patentinhaber: COURAGE + KHAZAKA ELECTRONIC GmbH, D-50829 Köln (DE)
(72) Erfinder: KHAZAKA, Gabriel, D-50859 Köln (DE)
(74) Vertreter: Dallmeyer, Georg, Dipl.-Ing.
(86) Internationale Anmeldenummer: EP9600749
(87) Internationale Veröffentlichungsnummer: WO9625884

(56) Entgegenhaltungen:
- WO-A-91/06242
- WO-A-93/14699
- DE-A- 3 213 944
- FR-A- 2 063 743
- US-A- 5 119 828

## Beschreibung

Die Erfindung betrifft eine Meßeinrichtung zur Bestimmung der Fettausscheidung der Haut nach dem Oberbegriff des Anspruchs 1.

Derartige Meßsysteme werden benötigt, um Umfang und Verteilung der Fettausscheidung der Haut meßtechnisch zu erfassen. Aus der US-A-5,119,828 ist die Verwendung einer mikroporösen hydrophoben fettaufnehmenden opaken Folie bekannt, deren Poren sich bei Hautkontakt mit Hautfett füllen können und dadurch die ursprünglich opake Folie für Licht transparent machen. Die Folie ist mit einem steifen Papier verklebt, das im Kontaktbereich der Folie mit der Haut geschwärzt ist. Ein solches Meßsystem hat den Nachteil, daß das die Folie aufnehmende Papier bei der Applikation verknickt, und daß dadurch die optische Auswertung des Hautabdrucks auf der Folie erschwert oder verfälscht wird.

Der Erfindung liegt die Aufgabe zugrunde, eine verbesserte Meßeinrichtung zur Bestimmung der Fettausscheidung der Haut zu schaffen, mit der die Reproduzierbarkeit der Meßwerte verbessert ist und die eine einfachere Auswertung ermöglicht.

Zur Lösung dieser Aufgabe dienen erfindungsgemäß die Merkmale des Anspruchs 1.

Die Erfindung sieht in vorteilhafter Weise vor, daß die fettaufnehmende Folie mit einem Träger im Randbereich verbunden ist, wobei der Träger in dem mit der Hautoberfläche in Kontakt zu bringenden Bereich der Folie ausgespart oder transparent ist. Eine derartige Meßeinrichtung gewährleistet, daß die fettaufnehmende Folie eingespannt ist und nach der Applikation im glatten Zustand ausgewertet werden kann. Insbesondere ist eine optische oder optoelektronische Auswertung im Durchlichtverfahren möglich, so daß die Auswertung insbesondere mit optoelektronischen Mitteln mit hoher Meßgenauigkeit durchgeführt werden kann. Dabei ist von Bedeutung, daß die Fläche der fettaufnehmenden Folie absolut eben ist, so daß sich bei der Auswertung keine Fokssierungsprobleme ergeben, die die Meßergebnisse verfälschen können.

Der Träger kann ringförmig gestaltet sein. Dabei kann der Träger aus zwei Ringen bestehen, die die fettaufnehmende Folie zwischen sich einspannen.

Der Träger kann aus einer transparenten, steifen Trägerfolie bestehen, die im Randbereich mit der fettaufnehmenden Folie auf der der Hautoberfläche abgewandten Seite der Folie verbunden ist.

Eine nichtklebende transparente Folie kann zwischen der fettaufnehmenden Folie und dem Träger angeordnet sein.

Eine solche Folie dient der Abstützung der fettaufnehmenden Folie.

Der Träger weist auf der der Hautoberfläche abgewandten Seite zumindest im Randbereich eine Klebefläche auf. Damit kann die Folie mit dem Träger an eine Einrichtung zum Auswerten der fettaufnehmenden Folie befestigt werden.

Auf der Klebefläche der Trägerfolie kann eine Schutzfolie, z.B. aus Silikonmaterial, angeordnet sein, wodurch die Klebefläche vor der Benutzung geschützt wird.

Vorzugsweise ist vorgesehen, daß die mindestens eine Folie im Randbereich mit Ultraschall mit dem Träger verschweißt ist. Das Verschweißen mit Ultraschall hat den Vorteil, daß kein Klebstoff die Eigenschaften der fettaufnehmenden Folie beeinflussen kann.

Der Träger kann mit der Klebefläche mit einem einer optischen oder optoelektronischen Auswerteeinrichtung angepaßten Trägerrahmen mit ausgespartem oder transparentem Mittelfeld aufgeklebt werden. Beispielsweise besteht die Möglichkeit, den Träger mit der fettaufnehmenden Folie auf einen Diarahmen aufzukleben und mit diesem Rahmen einer automatisierten Auswertung zuzuführen.

Im folgenden werden unter Bezugnahme auf die Zeichnungen Ausführungsbeispiele der Erfindung näher erläutert.

Es zeigen:
- Fig. 1: einen Querschnitt durch ein erstes Ausführungsbeispiel der Erfindung,
- Fig. 2: einen Teststreifen,
- Fig. 3: einen Querschnitt durch ein zweites Ausführungsbeispiel, und
- Fig. 4: einen Trägerrahmen für die Auswertung des Folienabdrucks.

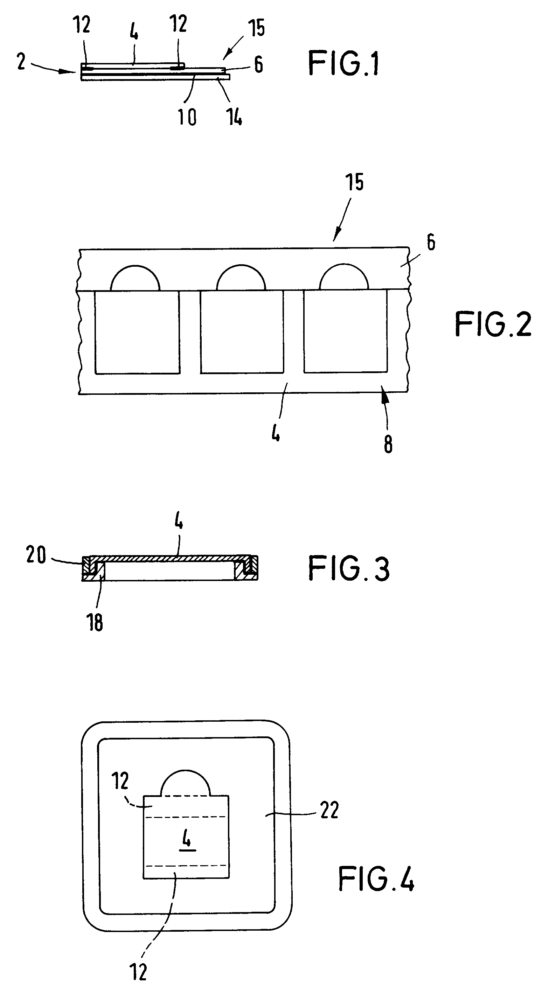

Die Meßeinrichtung zur Bestimmung der Fettausscheidung der Haut ist nach einem ersten Ausführungsbeispiel gemaß Fig. 1 mehrschichtig aufgebaut und besteht aus einer fettaufnehmenden äußeren Folie 4, die an zwei gegenüberliegenden Randbereichen 12 mit einer Tragerfolie 6 aus transparentem, steifem Material verbunden ist, wobei eine Schutzfolie 14 mit einer vorzugsweise an der Schutzfolie 14 angebrachten Klebeschicht 10 auf der der Folie 4 abgewandten Seite der Trägerfolie 6 angeordnet ist.

Die fettaufnehmende Folie 4 besteht beispielsweise aus einer mikroporösen, hydrophoben Polypropylenfolie mit einer Porösität zwischen 20 und 50 %, einer Dicke zwischen 20 und 30 µm und einer Porengröße zwischen 0,03 und 0,15 µm. Eine solche Folie weist eine gleichförmige Mikroporenverteilung auf und ist in ihrem Ursprungszustand opak. Bei Kontaktierung mit Fett füllen sich die Poren, wodurch die Folie an den entsprechenden Stellen transparent wird. Eine solche Folie eignet sich daher für vielfältige Untersuchungen hinsichtlich der Fettausscheidung der Haut, wenn die Folie in Kontakt mit der Hautoberfläche ggf. unter Aufbringung eines leichten Anpressdrucks gebracht wird.

Die Folie 4 ist in ihren Randbereichen 12 beispielsweise mit Ultraschall mit der Trägerfolie 6 verschweißt und wird von der steifen Trägerfolie 6 gespannt gehalten. Die Trägerfolie 6 ist in gewissem Umfang flexibel und erlaubt ein Anpressen der Folie 4 gegen die Hautoberfläche, ohne daß die Trägerfolie 6 selbst verknicken kann, so daß die Folie 4 auch zur Auswertung des Hautabdrucks gespannt gehalten werden kann, und sich keine Meßfehler aufgrund einer unebenen Folie 4 bei der Auswertung ergeben können.

Die Trägerfolie 6 kann in ihrem Mittelfeld auch ausgespart sein, wobei die Aussparung ein definiertes Meßfeld auf der Folie 4 begrenzt. In diesem Fall kann die Trägerfolie 6 auch aus lichtundurchlässigem Material bestehen.

Fig. 2 zeigt einen Teststreifen 8 mit mehreren Meßeinrichtungen zur Bestimmung der Fettausscheidung der Haut. Die Meßeinrichtungen sind dabei mit einem Stanzschnitt in einem entsprechend geschichteten Streifenmaterial zur Entnahme vorbereitet und können leicht von dem Teststreifen 8 abgelöst werden.

Fig. 3 zeigt ein zweites Ausführungsbeispiel, bei dem die fettaufnehmende Folie 4 zwischen zwei Ringen oder Rahmenteilen 18,20 eingespannt ist. Derartige Ringe halten die fettaufnehmende Folie 4 gespannt und können ebenfalls mit Hilfe einer Klebeschicht auf der der Folie 4 abgewandten Seite des Rings 18 auf einen Trägerrahmen 22 für eine erleichterte oder automatisierte Auswertung aufgeklebt werden. Die Meßeinrichtung mit den Rahmenteilen 18,20 und der Folie 4 kann selbstverständlich auch ohne Trägerrahmen 22 direkt optisch oder optoelektronisch ausgewertet werden.

Fig. 4 zeigt den Trägerrahmen 22 in Verbindung mit einer Meßeinrichtung gemäß den Fign. 1 und 2. Dabei wird die Meßeinrichtung mit Hilfe einer vorzugsweise am Trägerrahmen 22 vorhandenen Klebeschicht befestigt. Ein solcher Trägerrahmen 22 kann beispielsweise aus einem genormten Diarahmen bestehen.

## Patentansprüche

1. Meßeinrichtung zur Bestimmung der Fettausscheidung der Haut, mit einer mikroporösen, hydrophoben, fettaufnehmenden opaken Folie (4), die bei Hautkontakt das von der Hautoberfläche abgeschiedene Fett absorbieren kann, und deren Lichtdurchläßigkeit ein Maß für das aufgenommene Fett ist,
**dadurch gekennzeichnet**,
daß die fettaufnehmende Folie (4) mit einem Träger (2) im Randbereich verbunden ist, und daß der Träger (2) in dem mit der Hautoberfläche in Kontakt zu bringenden Bereich der Folie (4) ausgespart oder transparent ist.

2. Meßeinrichtung nach Anspruch 1, dadurch gekennzeichnet, daß der Träger (2) aus zwei Rahmenteilen (18,20) besteht, zwischen denen die fettaufnehmende Folie (4) eingespannt ist.

3. Meßeinrichtung nach Anspruch 1 oder 2, dadurch gekennzeichnet, daß der Träger (2) ringförmig ist.

4. Meßeinrichtung nach Anspruch 1 oder 2, dadurch gekennzeichnet, daß die fettaufnehmende Folie (4) auf der der Hautoberfläche abgewandten Seite mit mindestens einer den Träger (2) bildenden transparenten Trägerfolie (6) im Randbereich verbunden ist.

5. Meßeinrichtung nach einem der Ansprüche 1 bis 4, dadurch gekennzeichnet, daß eine nichtklebende transparente Folie zwischen der fettaufnehmenden Folie (4) und dem Träger (6) angeordnet ist.

6. Meßeinrichtung nach einem der Ansprüche 1 bis 5, dadurch gekennzeichnet, daß der Träger (6) auf der der Hautoberfläche abgewandten Seite zumindest im Randbereich eine Klebefläche (10) aufweist.

7. Meßeinrichtung nach Anspruch 4 bis 6, dadurch gekennzeichnet, daß die Trägerfolie (6) aus einer doppelseitig klebenden Folie besteht.

8. Meßeinrichtung nach einem der Ansprüche 4 bis 7, dadurch gekennzeichnet, daß auf der Trägerfolie (6) eine Schutzfolie (14) mit einer Klebefläche (10) angeordnet ist.

9. Meßeinrichtung nach einem der Ansprüche 1 bis 8, dadurch gekennzeichnet, daß die Folie (4) im Randbereich mit Ultraschall mit dem Träger (2,6) verschweißt sind.

10. Meßeinrichtung nach einem der Ansprüche 3 bis 9, dadurch gekennzeichnet, daß ein einer optischen oder optoelektronischen Auswerteeinrichtung angepaßter Trägerrahmen (22) mit ausgespartem oder transparentem Mittelfeld den Träger (2) aufnimmt.

## Claims

1. A measuring means for determining the secretion of sebum from the skin, comprising a microporous hydrophobic sebum absorbing opaque film (4) which is adapted to absorb sebum secreted by the skin surface when in contact therewith, the light permeability of which is a measure of the sebum absorbed,
characterized in
that the sebum absorbing film (4) is connected with a substrate (2) in the edge portion and that the substrate (2) is cut out or transparent in the area of the film (4) to be brought into contact with the skin surface.

2. The measuring means of claim 1, characterized in that the substrate (2) comprises two frame parts (18, 20), said sebum absorbing film (4) being tightened between said frame parts.

3. The measuring means of claim 1 or 2, characterized in that the substrate (2) is annular.

4. The measuring means of claim 1 or 2, characterized in that, on the side averted from the skin surface, the sebum absorbing film (4) is connected in the edge portion with at least one transparent substrate film (6) forming the substrate (2).

5. The measuring means of one of claims 1 to 4, characterized in that a non-adhesive transparent film is provided between the sebum absorbing film (4) and the substrate (6).

6. The measuring means of one of claims 1 to 5, characterized in that the substrate (6) has an adhesive area (10) at least in the edge portion on the side averted from the skin surface.

7. The measuring means of one of claims 4 to 6, characterized in that the substrate film (6) is a double-side adhesive film.

8. The measuring means of one of claims 4 to 7, characterized in that a protective film (14) with an adhesive surface (10) is provided on the substrate film (6).

9. The measuring means of one of claims 1 to 8, characterized in that the film (4) is ultrasonically welded to the substrate (2, 6) in the edge portion.

10. The measuring means of one of claims 3 to 9, characterized in that a support frame (22), adapted to an optical or opto-electronic evaluation means and having a cut out or transparent central part, receives the substrate (2).

## Revendications

1. Dispositif de mesure pour déterminer la sécrétion de sébum de la peau, comportant une feuille (4) opaque de prélèvement de sébum, hydrophobe, microporeuse , qui peut absorber au contact de la peau le sébum sécrété par la surface de la peau, et dont la transparence est une mesure pour le sébum prélevé,
caractérisé en ce que la feuille de prélèvement de sébum (4) est liée à un support (2) dans la zone de bordure, et en ce que le support (2) est évidé ou transparent dans la zone de la feuille (4) qui doit être amenée au contact de la surface de la peau.

2. Dispositif de mesure selon la revendication 1, caractérisé en ce que le support (2) se compose de deux parties de cadre (18, 20) entre lesquelles est serrée la feuille de prélèvement de sébum (4).

3. Dispositif de mesure selon la revendication 1 ou 2, caractérisé en ce que le support (2) a une forme d'anneau.

4. Dispositif de mesure selon la revendication 1 ou 2, caractérisé en ce que la feuille de prélèvement de sébum (4) est reliée, dans la zone de bordure du côté opposé à la surface de la peau, à au moins une feuille porteuse (6) transparente formant le support (2).

5. Dispositif de mesure selon l'une des revendications 1 à 4, caractérisé en ce qu'une feuille transparente non adhésive est disposée entre la feuille de prélèvement de sébum (4) et le support (6).

6. Dispositif de mesure selon l'une des revendications 1 à 5, caractérisé en ce que le support (6) comporte une surface adhésive (10) au moins dans la zone de bordure du côté opposé à la surface de la peau.

7. Dispositif de mesure selon revendication 4 à 6, caractérisé en ce que la feuille porteuse (6) se compose d'une feuille adhésive sur les deux faces.

8. Dispositif de mesure selon l'une des revendications 4 à 7, caractérisé en ce qu'une feuille de protection (14) comportant une surface adhésive (10) est disposée sur la feuille porteuse (6).

9. Dispositif de mesure selon l'une des revendications 1 à 8, caractérisé en ce que, dans la zone de bordure, les feuilles (4) sont soudées au support (2, 6) par ultrasons.

10. Dispositif de mesure selon l'une des revendications 3 à 9, caractérisé en ce qu'un cadre porteur (22), adapté à un dispositif d'évaluation optique ou optoélectronique et comportant une zone centrale évidée ou transparente, sert de logement au support (2).
